# EUROPEAN PATENT SPECIFICATION

(11) **EP 3 038 676 B1**
(45) Date of publication and mention of the grant of the patent: **04.11.2026**
(21) Application number: 14755665.8
(22) Date of filing: 26.08.2014
(51) Int. Cl.: A61M 5/31, A61M 5/32, A61M 5/24, A61M 5/315

(54) **ASSEMBLY FOR DRUG DELIVERY DEVICE, DRUG DELIVERY DEVICE COMPRISING SUCH ASSEMBLY AND METHOD FOR PRODUCING THE ASSEMBLY**
ANORDNUNG FÜR ARZNEIMITTELABGABEVORRICHTUNG, ARZNEIMITTELABGABEVORRICHTUNG UND VERFAHREN ZUM HERSTELLEN EINER SOLCHEN ANORDNUNG
ENSEMBLE POUR DISPOSITIF D'ADMINISTRATION DE MÉDICAMENT, LEDIT DISPOSITIF ET PROCÉDÉ DE PRODUCTION D'UN TEL ENSEMBLE

(30) Priority: 29.08.2013 EP 13182221
(43) Date of publication of application: 06.07.2016
(73) Proprietor: Sanofi-Aventis Deutschland GmbH, 65929 Frankfurt am Main (DE)
(72) Inventor: PLUMPTRE, David Aubrey, Droitwich Spa, Worcestershire WR9 7RQ (GB)
(74) Representative: Zwicker, Jörk
(86) International application number: PCT/EP2014/068023
(87) International publication number: WO 2015/028441

(56) References cited:
- EP-B1- 1 603 611
- WO-A1-2008/128645
- WO-A1-2010/112558
- WO-A1-88/08725
- CN-Y- 201 097 144
- DE-A1- 1 561 832
- US-A1- 2004 015 038
- US-A1- 2007 265 579

## Description

The present invention relates to an assembly for a drug delivery device. Furthermore, the present invention relates to a drug delivery device comprising the assembly and to a method of producing the assembly.

Drug delivery devices, in particular pen-type drug delivery devices, have application where regular injection by persons without formal medical training occurs. This may be increasingly common among patients having diabetes where self-treatment enables such patients to conduct effective management of their disease. In practice, such a drug delivery device allows a user to dispense a number of user variable doses or fixed doses of a medicament. Assemblies for drug delivery devices are known from WO 88/08725 A1, WO 2008/128645 A1, EP 1 603 611 A1 and WO 2010/112558 A1. In particular, WO 88/08725 A1 discloses an assembly for a drug delivery device having a metal element and a plastic element, the metal element having an aperture and the metal and plastic element being coupled to each other.

It is an object of the present invention to provide an assembly for a drug delivery device having improved properties. In particular, it is an object to provide an assembly for a drug delivery device with a sophisticated design.

In accordance with a first aspect of the invention an assembly for a drug delivery device as specified in claim 1 is provided.

In particular, the outer surface may provide a supporting surface for a hand of a user, e.g. a user of the drug delivery device, during a use of the drug delivery device.. In particular, the metal component and the plastic component are rotationally and axially fixed with respect to each other

The advantage of an assembly wherein a metal component forms an outer surface is that a user associates a metal surface with high quality. In particular, a metal surface has pleasant haptical properties to a user. For example a metal surface may feel smooth and cool.

Furthermore, a metal surface may look more precious than a plastic surface. Yet, due to the plastic element, the assembly may have less weight than an assembly which is completely produced of metal. Thereby, a drug delivery device comprising the assembly is easier to handle by a user. Furthermore, the material costs are kept low without losing functional properties. In particular, the plastic element may improve the stability of the metal element. Due to the plastic element, the metal element may withstand forces acting on the metal element during an assembly of the drug delivery device. With the plastic element supporting the metal element, the metal element may be dimensionally stable. Additionally, the plastic element may be moulded with features that attach to other existing inner plastic components. Furthermore, the plastic element and/or the metal element may comprise other features, such as one or more stop features, for example an '80Units stop' feature, that is/are configured or required to prevent the user from setting a dose greater than a pre-determined maximum dose. In particular, such a stop feature may be configured so as to provide a defined rotational and/or axial stop for a dialing member of a drug delivery device, such as a dial sleeve or whatsoever. In this way, e.g. an axial travel of such a dialing member which corresponds to a dialed dose of a medicament can be delimited such that dose dialing can be restricted to a predetermined maximum dose. For example, in an end position of a dial sleeve with respect to the assembly a stop feature on the dial sleeve may be engaged with said stop feature of the metal element or the plastic element of the assembly, thereby determining a maximum set dose.

For example, the assembly may form an outer housing part or a cap for a drug delivery device. According to a conceivable embodiment, both a housing part and a cap for a drug delivery device may be formed by a respective assembly. In particular, the assembly may be a part for a drug delivery device which is often touched by a user. In case that the plastic element is a housing part, the plastic element may be attached to an inner housing body. In particular, the inner housing body may comprise retention features that may engage with the plastic element.

A functional unit may be an assembly which once assembled acts as one part. The metal element and the plastic element may be permanently or releasably coupled.

According to one embodiment, the length of the metal element and the plastic element may be essentially equal. According to another embodiment, the length of the plastic element may be less than the length of the metal element. For example, the length of the plastic element may be between one fourth and one half of the length of the metal element. Thereby, the plastic element may be easier and cheaper to produce.

The metal element may comprise aluminium or another metal material. An outer surface of the metal element may be anodised. The anodising provides a high quality and hard wearing exterior surface to the metal element. Furthermore, it enables it to be given a variety of metallic colours. In a further embodiment, the metal element may be galvanised. Alternatively, the metal element may be surface treated by brushing. Furthermore, the metal element may be varnished with a clear coating material.

The metal element may essentially comprise the shape of a sleeve. A wall thickness of the metal element may be between 0.3 mm and 0.6 mm. Preferably, the wall thickness of the metal element may be 0.4 mm. Thereby, the metal element may comprise a sufficient stability while having a low weight.

The metal element may be manufactured by deep drawing. Thereby, the metal element may comprise an end face which is inclined with respect to a longitudinal axis of the metal element. For example, the end face may be inclined about 60° to 80° with respect to the longitudinal axis of the metal element.

For example, the plastic element may comprise Polyoxymethylen or another plastic material. The plastic element may be produced by mold flowing.

The shape of the plastic element and the metal element may be adapted to each other. In particular, an outer surface of the plastic element may be adapted to an inner surface of the metal element in a large part. This means that only a small part of the outer surface of the plastic element, for example a part which is necessary to align the plastic element and the metal element with respect to each other, is not in contact with an inner surface of the metal element.

According to one embodiment, the metal element comprises one or more radial lugs. The metal element and the plastic element may be held together by the radial lug of the metal extending towards the plastic element. In particular, the radial lug may engage the plastic element. Thereby, an axial and rotational position of the metal element and the plastic element with respect to each other may be maintained. In particular, the metal element may be connected to the plastic element by means of the radial lug. The lug features are preferably formed by punching. The metal element and the plastic element may be connected by reshaping the metal element.

According to one embodiment, the metal element comprises one or more additional lug features, which are configured to serve as a stop feature, for example as an '80Units stop' feature, instead of the plastic element. The additional lug features may be folded into the metal element. Preferably, the additional lug features do not contact the plastic element.

In particular, the metal element and the plastic element may each comprise an aperture which are aligned with each other. The aperture in the metal element may be cut out by blanking. The aperture in the plastic element may be provided during the moulding process.

According to one embodiment, the radial lug of the metal element may be located at the aperture. In order to secure the metal element and the plastic element with respect to each other, the radial lug is folded into the aperture.

For example, the further component may stick through the aperture of the metal element. Thereby, the metal element and the plastic element may be held in a fixed position. Furthermore, the further component may be clipped in the plastic element.

According to one embodiment, the metal element may comprise an aperture. The plastic element may be made from a transparent material. In this embodiment, the window component may be comprised by the plastic element. In particular, no further window component is necessary.

According to one embodiment, the plastic element may comprise a protrusion. The protrusion may be located at an axial end of the plastic element. The protrusion may extend in a radial outward direction. The metal element may extend over the plastic element until it abuts the protrusion. Thereby, an axial position of the metal element with respect to the plastic element may be defined. Furthermore, the protrusion of the plastic element may comprise a nose which extends in a longitudinal direction. The nose may be configured to engage with a corresponding recess of the metal element. Thereby, a rotational position of the metal element with respect to the plastic element may be defined.

According to one embodiment, the metal element and the plastic element may be secured with respect to each other by press fitting.

According to another aspect of the invention, a drug delivery device is provided. The drug delivery device comprise an assembly as described above. The drug delivery device may comprise further elements, such as a drive assembly, a cartridge holder and a cartridge. The drug delivery device has a weight of less than 30 g. For example, the drug delivery device may have a weight of less than 30 g containing a cartridge which is full with a medicament.

Alternatively, the drug delivery device may have a weight of less than 30 g containing an empty cartridge. This may be achieved by the combination of a metal element and a plastic element in one functional unit. A drug delivery device having a weight of less than 30 g has the advantage that it is easy to handle by a user.

The drug delivery device may be an injection device. The drug delivery device may be a pen-type device. The drug delivery device may be a fixed dose device such that the amount of medication which is delivered during one dispense operation is predetermined. In particular, a user may not be enabled to vary the size of the dose. Alternatively, the drug delivery device may be a variable dose device such that the amount of medication which is delivered during one dispense operation may be adjusted by a user. The drug delivery device may be configured for multiple dose applications. The medication may be delivered to a user by means of a needle. The device may be delivered to a user in a fully assembled condition ready for use. The drug delivery device may be a reusable device. Alternatively, the drug delivery device may be a disposable device. The term "disposable" means that the drug delivery device cannot be reused after an available amount of medication has been delivered from the drug delivery device. The drug delivery device may be configured to deliver a liquid medication. The medication may be, for example, insulin.

According to a further aspect of the invention, a method of producing an assembly for a drug delivery device in accordance with the first aspect of the invention is provided. The method comprises the providing of a metal element and a plastic element. The metal element and the plastic element may have the shape of a sleeve. During assembling, the metal element is slid over the plastic element. In particular, the metal element is slid over the plastic element in a longitudinal direction.

The term "medication", as used herein, preferably means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, an enzyme, an antibody or a fragment thereof, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exendin-3 or exendin-4 or an analogue or derivative of exendin-3 or exendin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

Exendin-4 for example means Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-lle-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
H-(Lys)5-des Pro36, des Pro37 Exendin-4(1-39)-NH2,
des Pro36 Exendin-4(1-39),
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(O2)25, Asp28] Exendin-4(1-39),
des Pro36 [Met(O)14 Trp(O2)25, IsoAsp28] Exendin-4(1-39); or
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(O2)25, Asp28] Exendin-4(1-39),
des Pro36 [Met(O)14 Trp(O2)25, IsoAsp28] Exendin-4(1-39),
wherein the group -Lys6-NH2 may be bound to the C-terminus of the Exendin-4 derivative;
or an Exendin-4 derivative of the sequence
des Pro36 Exendin-4(1-39)-Lys6-NH2 (AVE0010),
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(O2)25, Asp28] Exendin-4(1-39)-Lys6-NH2,
H-des Asp28 Pro36, Pro37, Pro38 [Trp(O2)25] Exendin-4(1-39)-NH2,
H-(Lys)6-des Pro36, Pro37, Pro38 [Trp(O2)25, Asp28] Exendin-4(1-39)-NH2,
H-Asn-(Glu)5-des Pro36, Pro37, Pro38 [Trp(O2)25, Asp28] Exendin-4(1-39)-NH2,
des Pro36, Pro37, Pro38 [Trp(O2)25, Asp28] Exendin-4(1-39)-(Lys)6-NH2,
H-(Lys)6-des Pro36, Pro37, Pro38 [Trp(O2)25, Asp28] Exendin-4(1-39)-(Lys)6-NH2,
H-Asn-(Glu)5-des Pro36, Pro37, Pro38 [Trp(O2)25, Asp28] Exendin-4(1-39)-(Lys)6-NH2,
H-(Lys)6-des Pro36 [Met(O)14, Asp28] Exendin-4(1-39)-Lys6-NH2,
des Met(O)14 Asp28 Pro36, Pro37, Pro38 Exendin-4(1-39)-NH2,
H-(Lys)6-desPro36, Pro37, Pro38 [Met(O)14, Asp28] Exendin-4(1-39)-NH2,
H-Asn-(Glu)5-des Pro36, Pro37, Pro38 [Met(O)14, Asp28] Exendin-4(1-39)-NH2,
des Pro36, Pro37, Pro38 [Met(O)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(O2)25, Asp28] Exendin-4(1-39)-Lys6-NH2,
H-des Asp28 Pro36, Pro37, Pro38 [Met(O)14, Trp(O2)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(O)14, Trp(O2)25, Asp28] Exendin-4(1-39)-NH2,
des Pro36, Pro37, Pro38 [Met(O)14, Trp(O2)25, Asp28] Exendin-4(1-39)-(Lys)6-NH2,
H-(Lys)6-des Pro36, Pro37, Pro38 [Met(O)14, Trp(O2)25, Asp28] Exendin-4(S1-39)-(Lys)6-NH2,
H-Asn-(Glu)5-des Pro36, Pro37, Pro38 [Met(O)14, Trp(O2)25, Asp28] Exendin-4(1-39)-(Lys)6-NH2;
or a pharmaceutically acceptable salt or solvate of any one of the afore-mentioned Exendin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies are globular plasma proteins (~150 kDa) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two β sheets create a "sandwich" shape, held together by interactions between conserved cysteines and other charged amino acids.

There are five types of mammalian Ig heavy chain denoted by α, δ, ε, γ, and µ. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (C_{H}) and the variable region (V_{H}). In one species, the constant region is essentially identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals, there are two types of immunoglobulin light chain denoted by λ and κ. A light chain has two successive domains: one constant domain (CL) and one variable domain (VL). The approximate length of a light chain is 211 to 217 amino acids. Each antibody contains two light chains that are always identical; only one type of light chain, κ or λ, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

A non-limiting, exemplary embodiment of the invention will now be described with reference to the accompanying drawings, in which:
- Figure 1: shows a drug delivery device with a cap attached in accordance with the present invention;
- Figure 2: shows the drug delivery device of Figure 1 with the cap removed and a dose of 79 units dialed;
- Figure 3: shows in an exploded view the components of the drug delivery device of Figure 1;
- Figure 4: shows the outer body of the drug delivery device of Figure 1;
- Figure 5a: shows the inner body of the drug delivery device of Figure 1;
- Figure 5b: shows a detail of the inner body of Figure 5a;
- Figure 6: shows the cartridge holder of the drug delivery device of Figure 1;
- Figure 7a: shows a first display member component of the drug delivery device of Figure 1;
- Figure 7b: shows a detail of the first display member of Figure 7a;
- Figure 8: shows a second display member component of the drug delivery device of Figure 1;
- Figure 9: shows a first driver component of the drug delivery device of Figure 1;
- Figure 10: shows a second driver component of the drug delivery device of Figure 1;
- Figure 11: shows a third driver component of the drug delivery device of Figure 1;
- Figure 12: shows the last dose nut of the drug delivery device of Figure 1;
- Figure 13: shows a clutch component of the drug delivery device of Figure 1;
- Figure 14: shows a first clicker component of the drug delivery device of Figure 1;
- Figure 15: shows a second clicker component of the drug delivery device of Figure 1;
- Figure 16: shows the button of the drug delivery device of Figure 1;
- Figure 17: shows a cut-away view of the proximal part of the drug delivery device of Figure 1 in a zero unit position with the button released;
- Figure 18: shows a cut-away view of the proximal part of the drug delivery device of Figure 1 in a position with some units dialed;
- Figure 19: shows a cut-away view of the proximal part of the drug delivery device of Figure 1 in a zero unit position with the button pressed;
- Figure 20a: shows a plastic element of the outer housing part;
- Figure 20b: shows the plastic element and a metal element of the outer housing part during assembling;
- Figure 20c: shows the plastic element and the metal element of the outer housing part in an assembled state;
- Figure 21a: shows a plastic element of a cap;
- Figure 21b: shows the plastic element and a metal element of the cap;
- Figure 21c: shows a clip element being attached to the cap;
- Figure 22a: shows a snap feature of the inner housing;
- Figure 22b: shows the outer housing part being coupled to the inner housing;
- Figure 23a: shows a metal element of an outer housing part comprising a lug feature;
- Figure 23b: shows an assembly comprising a metal element comprising a lug feature and a plastic element in a sectional view;
- Figure 24a: shows a metal element of an outer housing part;
- Figure 24b: shows an assembly comprising a metal element and a plastic element in a sectional view;
- Figure 25a: shows a sectional view of a part of the outer housing part comprising a metal element and a plastic element;
- Figure 25b: shows a further sectional view of the outer housing part of Figure 25A;
- Figure 26: shows an '80Units stop' feature which is arranged on the metal element of the outer housing part;
- Figure 27: shows an assembly comprising the metal element of Figure 26.

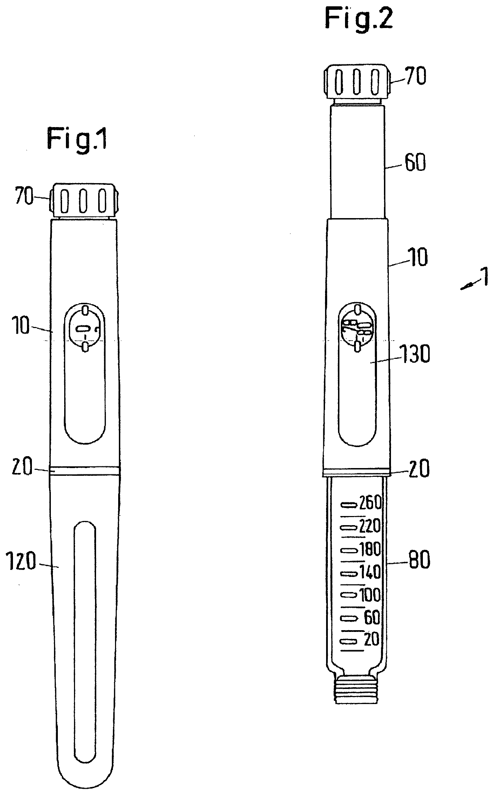

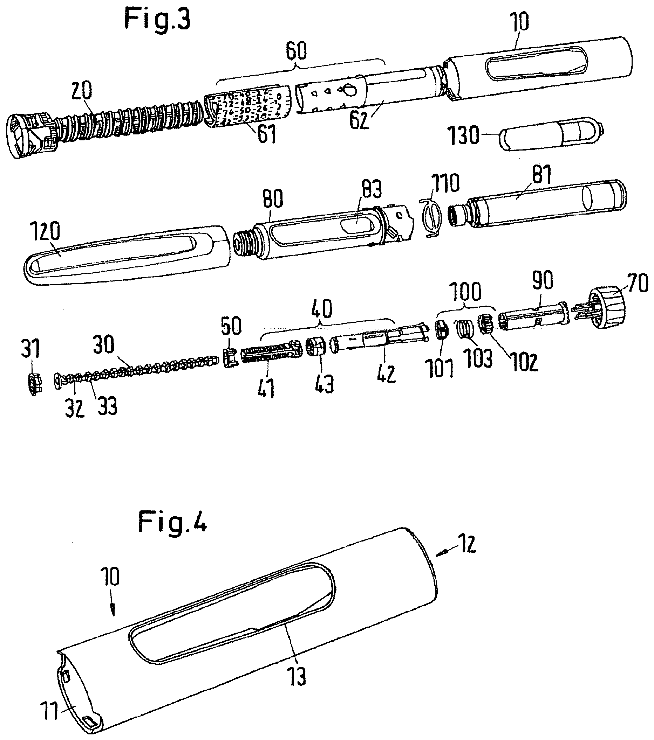

Figures 1 and 2 show a drug delivery device 1 in the form of an injection pen. The device has a distal end (lower end in Figure 1) and a proximal end (upper end in Figure 1). The component parts of the drug delivery device 1 are shown in Figure 3 in more detail. The drug delivery device 1 comprises an outer housing part 10, an inner body 20, a piston rod 30, a driver 40, a nut 50, a display member 60, a button 70, a cartridge holder 80 for receiving a cartridge 81, a clutch 90, a clicker 100, a spring 110, a cap 120 and a window insert 130. A needle arrangement (not shown) comprising a needle hub and a needle cover may be provided as additional components, which can be exchanged as explained above. The piston rod 30 comprises a bearing 31. The driver comprises a distal driver part 41, a proximal driver part 42 and a coupler 43. The display member 60 comprises a number sleeve 61 and a dial sleeve 62. The clicker comprises a distal clicker part 101, a proximal clicker part 102 and a spring 103.

The outer housing part 10, which is shown in Figure 4, is a generally tubular element having a distal part 11 for attaching the inner body 20 and a proximal part, which is provided with a rotational hard stop 12 on its inner surface (not shown) which contact mating faces of the display member 60 when the maximum units (in this example 80U) stop is engaged. The end face also serves as the end of dose dispense stop for the button 70, and the bore in the end face centers the display member 60 during both dialing and dispense. An aperture 13 is provided for receiving window insert 130. The outer body 10 provides the user with a surface to grip and react against during dispense.

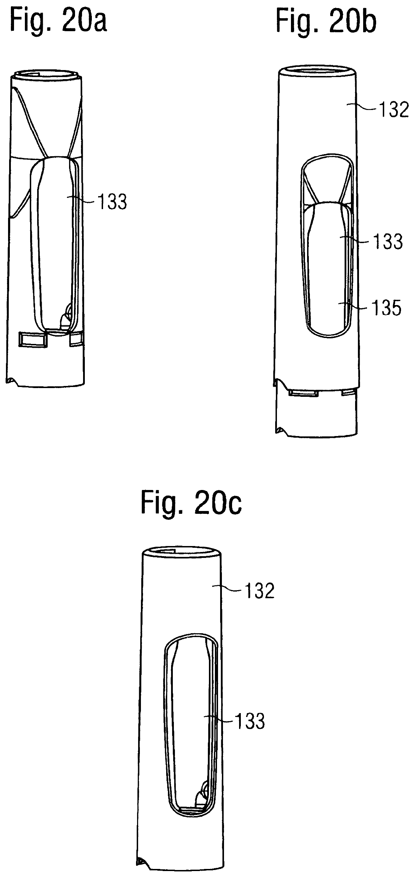

The outer housing part 10 comprises a metal element 132 and a plastic element 133. These elements are shown for example in Figures 20a to 20c. Figure 20a shows the plastic element 133 of the outer housing part 10. The plastic element 133 is produced by mold flowing or injection molding. The plastic element 133 may be made of Polyoxymethylen (POM) or of another plastic material. Figure 20b shows the plastic element 133 and the metal element 132 of the outer housing part 10 during assembling. In particular, the metal element 132 is slid over the plastic element 133. For example, the metal element 132 comprises a stop face (not shown), wherein the metal element 132 can be slid over the plastic element 133 until the stop face of the metal element 132 abuts the plastic element 133. The stop face of the metal element 132 may be produced by beading one end of the metal element 132. In particular, the beaded end forms the stop face of the metal element 132. The metal element 132 is produced by deep drawing. The metal element 132 may be made of aluminium or of another metal material.

The plastic element 133 is coupled to the inner housing 20. The inner housing 20 comprises snap features 139. The snap features 139 interact with the plastic element 133, thereby fixing the plastic element with respect to the inner housing 20.

The metal element 132 comprises an aperture 135, which is configured for receiving the window insert 130.

The outer surface of the plastic element 133 is adapted to the inner surface of the metal element 132. Thereby, the plastic element 133 and the metal element 132 may be accurately fitted together. Each of the plastic element 133 and the metal element 132 may have the form of a sleeve.

The plastic element 133 and the metal element 132 are fixedly coupled. In particular, the plastic element 133 and the metal element 132 are fixed together in an axial and rotational direction.

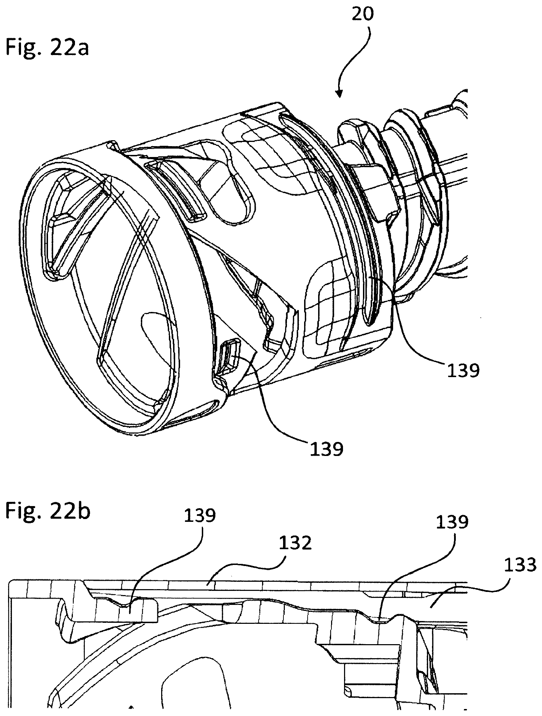

Figures 22a and 22b show snap features 139 of the inner housing 20. By means of the snap features 139, the outer housing part 10 is coupled to the inner housing 20. In particular, the snap features 139 are engaged with the plastic element 133 of the outer housing part 10.

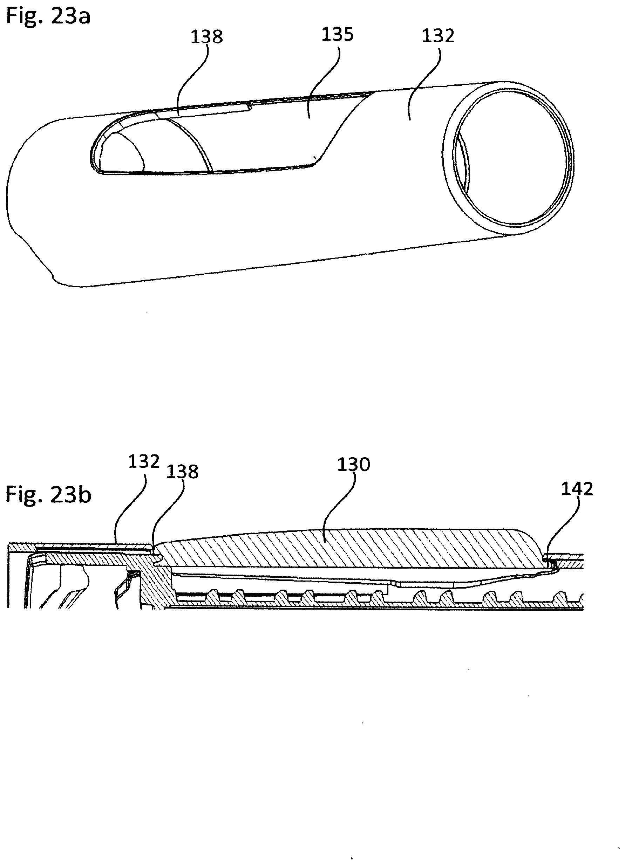

Figure 23a shows the metal element 132 of the outer housing part 10. The metal element 132 comprises a radial lug 138. The lug feature 138 may comprise one extended lug or a series of smaller lugs. The radial lug 138 is folded into the aperture 135. The radial lug 138 may be folded along a full perimeter of the aperture 135 or locally as shown. By means of the radial lug 138, the metal element 132 is secured to the plastic element 133, as shown in Figure 23b. In particular, the radial lug 138 engages with the plastic element 133. Thereby an axial and rotational position of the metal element 132 with respect to the plastic element 133 is maintained.

In the design shown in Figure 23a, the radial lug 138 is folded into the aperture 135 to a greater depth at one end of the metal element 132, but is not folded into the aperture 135 at the other end. By the radial lug 138 being folded into the aperture 135 to a greater depth at one end of the metal element 135 so that the edge folded down conforms to a plain undrafted diameter, assembling the metal element to the plastic element is made easier as the proximal end of the plastic element just has to assemble past this plain undrafted diameter. Due to the radial lug feature 138 being not present at the other end, a tab 142 (see Figure 23b) of the window insert 130 may be inserted through the aperture 135 under the outer housing part 10. Thereby, the window insert 130 may be secured to the assembly.

The plastic element 133 is configured to support an underside of the window insert 130. In particular, the window insert 130 lies on the plastic element 133. Thereby, a risk of scratching a printed lower surface of the window insert 130 is minimized.

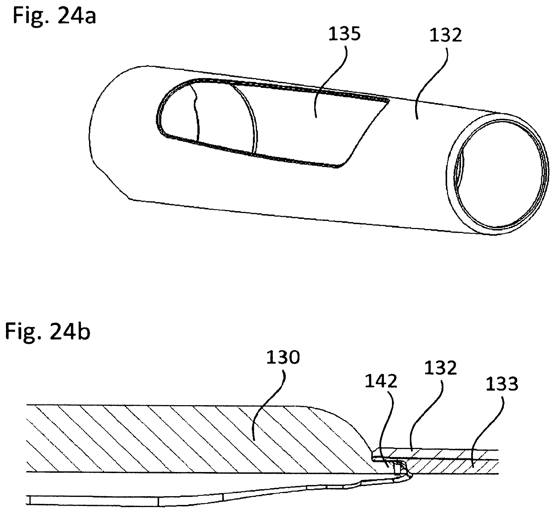

As it is intricate in practice to achieve a lug feature folded down to different depths around the aperture 135 in the metal element 132, a further embodiment as shown in Figures 24a and 24b omits the lug feature completely, and the metal element 132 just has a plain aperture cut into the metal element 132.

In this instance any rotational forces applied to the metal element 132 are transferred via the window insert 130 to the plastic element 133.

The metal element 132 and the plastic element 133 may be secured with respect to each other by the window insert 130, which is inserted through the aperture 135, as shown in Figure 24b. In particular, as shown in Figure 24a, the metal element 132 may be produced without any lug feature. By the window insert 130 extending through the aperture 135, the metal element 132 and the plastic element 133 may be axially and rotationally fixed with respect to each other.

The embodiment according to Figures 24a and 24b has the advantage that it is simpler to manufacture without the folded lug feature 138.

The plastic element 133 and the metal element 132 may additionally be coupled to each other by press fitting. Additionally or alternatively to press fitting, one or both of the plastic element 133 and the metal element 132 may comprise a feature which may fix the elements together by snap fitting. The plastic element 133 and the metal element 132 may additionally be fixed together by bonding with an adhesive or via application of heat to activate a bonding agent or to fuse the parts together.

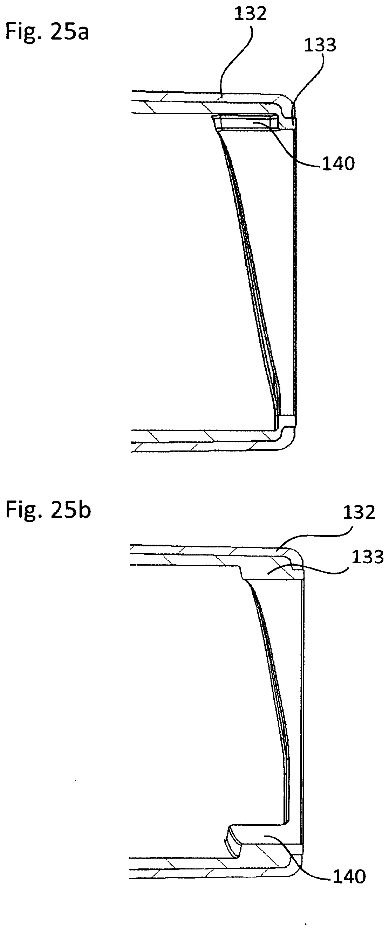

Additionally or alternatively to the press fitting, to the snap fitting or to the bonding with an adhesive or via application of heat, the plastic element 133 may comprise a protrusion (not shown). The protrusion may be located at an axial end of the plastic element 133. The protrusion may extend in a radial outward direction. The metal element 132 may extend over the plastic element 133 until it abuts the protrusion. Thereby, an axial position of the metal element 132 with respect to the plastic element 133 may be defined. Furthermore, the protrusion of the plastic element 133 may comprise a nose which extends in a longitudinal direction. The nose may be configured to engage with a corresponding recess of the metal element 132. Thereby, a rotational position of the metal element 132 with respect to the plastic element 132 may be defined. There may be a plurality of nose features to improve the rotational engagement. Additionally or alternatively the metal element may have a flange at the proximal end which partially encloses the plastic element so as to restrain it axially, see Fig 25a and 25b.

The plastic element 133 and the metal element 132 form a functional unit. The plastic element 133 and the metal element 132 may be releasably or permanently coupled to each other.

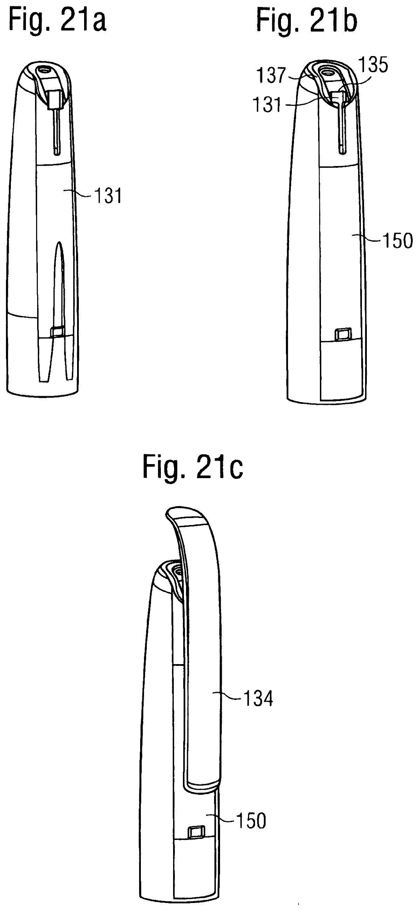

The cap 120 comprises, as does the outer housing part 10, a metal element 150 and a plastic element 131. The metal element 150 and the plastic element 131 of the cap are shown in Figures 21a to 21c. In particular, Figure 21a shows a plastic element 131 of the cap 120. Figure 21b shows the metal element 150 attached to the plastic element 131 of the cap 120. The metal element 150 and the plastic element 131 of the cap 120 are similar to the metal element 132 and the plastic element 133 of the outer housing part 10. Therefore, it is referred to the description above.

Yet, the plastic element 131 and the metal element 150 of the cap 120 have a slightly different shape than the corresponding elements of the outer housing part 10. In particular, the metal element 150 has an end face 137, which is inclined to a longitudinal axis of the assembly. The end face 137 may be inclined to a longitudinal axis of the cap for about 60° to 80°. The end face 137 is inclined for design reasons. Furthermore, the cap 120 comprises a clip element 134. The clip element 134 is inserted into an aperture 135 of the cap 120. The clip element 134 is fixed to the cap 120 for example by snap fitting. The clip element 134 being attached to the cap 120 is shown in Figure 21c. The clip element 134 may be configured to hold the metal element 130 and the plastic element 131 in a fixed position with respect to each other.

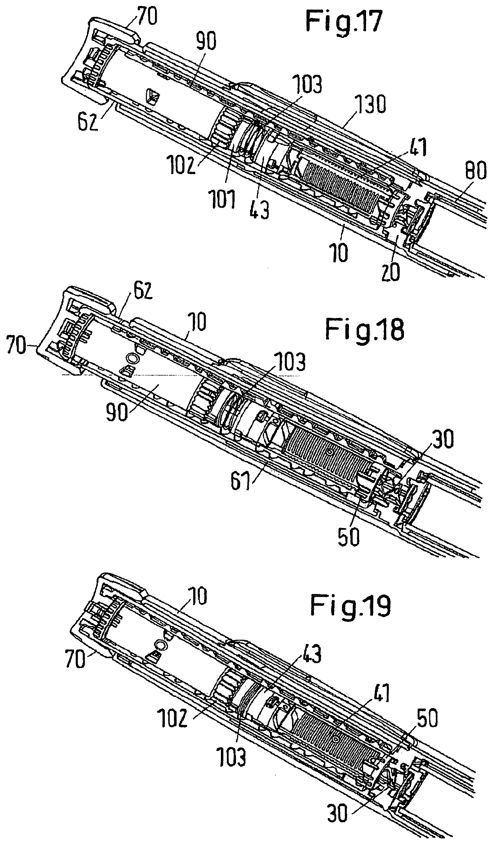

The inner body 20 is a generally tubular element having different diameter regions. As can be seen in Figures 17 to 19, the inner body 20 is received in the outer body 10 and permanently fixed therein to prevent any relative movement of the inner body 20 with respect to the outer body 10. The inner body has the functions to house the drive mechanism within, guiding the clickers and the last dose nut 50 via internal splines, to provide an internal thread through which the piston rod 30 (lead screw) is driven, to support and guide the number sleeve 61 and the dial sleeve 62 on an external thread form, to secure the cartridge holder 80 and to secure the outer body 10 and the window insert 130.

The outermost diameter of the inner body 20 also forms part of the visual design and remains visible when the cap 120 is secured to the cartridge holder 80 as a ring separating the cap 120 from the outer body 10. This visible ring also has depressions which align with the cap snap features on the cartridge holder 80 to indicate that the cartridge holder has been correctly fitted.

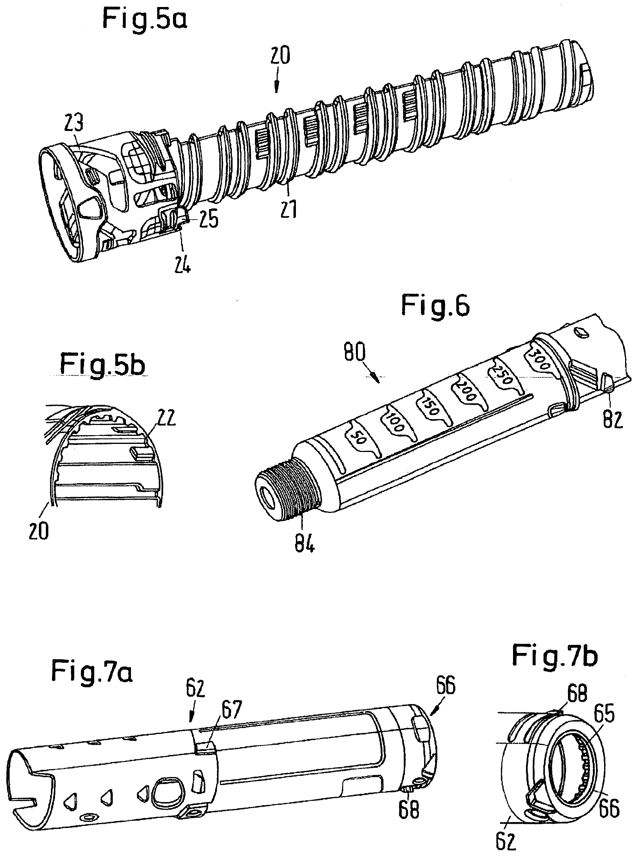

An external thread 21 is provided on the outer surface of the inner body 20. Further, splines 22 (Figure 5b) are provided on the inner surface of the inner body 20. These internal splines 22 guide the clicker 100 axially during both dialing and dispense and also prevent the last dose nut 50 from rotating. Some of the splines may be wider to ensure correct rotational assembly of the internal components, and these wider splines may have a stepped entry and angled surface to encourage the last dose nut 50 to rotate up against the stop face on the distal drive sleeve 41 during assembly. At the open end shown in Figure 5b there are an additional short splines which together with the alternating long splines 22 are used to rotationally lock the button 70 (dose dial grip) at the end of dispense and serve to increase the strength of the 0U dial stop when the button 70 is depressed. This is achieved by engagement with male spline features on the clutch component 90.

Bayonet features 23 guide the cartridge holder 80 into the mechanism during cartridge replacement, compressing the cartridge bias spring 110, and then back off the cartridge holder 80 a small distance in order to reduce axial play in the mechanism. Snap features inside the inner body 20 lock the cartridge holder 80 rotationally when it has been correctly fitted. The profile of these snaps aims to prevent the user from partially fitting the cartridge holder 80, the cartridge bias spring 110 ejecting the cartridge holder 80 if the snaps have not at least started to engage. A window retention nose 24 retains the window insert 130 when the outer body 10 and window insert 130 assembly is axially inserted onto the inner body 20. Two diametrically opposite stop faces 25 define the rotational end position for the number sleeve 61. This end position is the end of dose detent position for the minimum dose (0U).

The piston rod 30 is an elongate element having two external threads 32, 33 with opposite hand which overlap each other. One of these threads 32 engages the inner thread of the inner body 20. A disk-like bearing 31 is provided at the distal end of the piston rod 30. The bearing 31 may be a separate component as shown in Figure 3 or may be attached to the piston rod 30 as a one-piece component via a predetermined breaking point.

The piston rod 30 transfers the dispense load from the driver 40 to the bearing 31, creating a mechanical advantage greater than 1:1 by converting the torque generated on the piston rod 30 by the driver 40 thread interface into additional axial load as the piston rod passes through the thread in the inner body 20. The piston rod 30 is reset by pressing on the bearing 31 and this in turn rotates the piston rod back into the inner body 20. This disengages and then rotates the distal drive sleeve 41, resetting the last dose nut 50 back to its starting position on the distal drive sleeve 41.

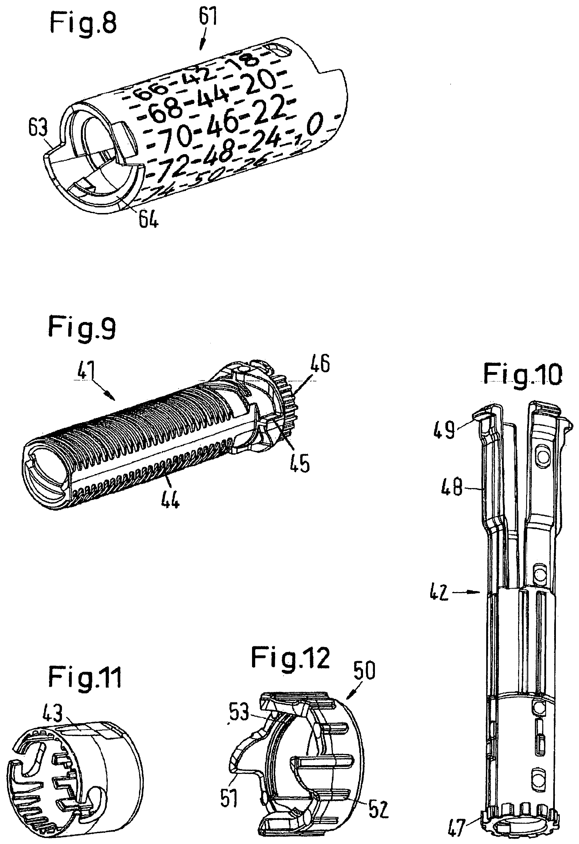

The driver 40 is a generally tubular element having in the embodiment shown in the Figures three components which are depicted in Figures 9 to 11 in more detail.

The distal drive sleeve 41 engages with the piston rod thread 33 to drive the piston rod 30 through the inner body 20 during dose delivery. The distal drive sleeve 41 is also permanently connected to the coupler 43 which in turn is releasably engaged through reset clutch features to the proximal drive sleeve 42. The two halves of the drive sleeve are rotationally and axially connected during dialing and dispense, but are de-coupled rotationally during device reset so that they can rotate relative to each other.

The external thread 44 engages with the last dose nut 50. The thread form has three stages, a shallow first stage (left hand side in Figure 9) over which the nut 50 travels to count the majority of the units dialed, a fast stage over which the last dose nut moves rapidly axially prior to engaging the stop faces, and a final shallow section which ensures that when the stop faces have engaged, the axial restraint on the nut 50 extends over a reasonable length of thread form. Four equi-spaced stop faces 45 engage with mating stop faces 51 on the last dose nut 50 to limit the number of units that can be dialed. Splines 46 are provided at the proximal end of distal drive sleeve 41 to transfer torque from or to the coupler 43, which may be snapped on the distal drive sleeve 41.

The proximal drive sleeve 42 shown in Figure 10 supports the clicker components 100 and the clutch 90 and transfers rotational movement from the dose button 90 to the coupler 42 and distal drive sleeve 41.

Teeth features 47 located at the distal end of proximal drive sleeve 42 engage with the reset clutch features on the coupler 43 to connect both halves of the drive sleeve during dialing and dispense. During reset these teeth 47 disengage.

Several splines are provided on the outer surface of proximal drive sleeve 42 engaging with distal clicker part 101, preventing relative rotation during dialing and dispense. Further splines, which are located in the middle region of proximal drive sleeve 42, engage with the clutch 90 component. They may be arranged to be non-rotationally symmetric so that the various clicker components cannot be assembled accidentally upside down.

The proximal portion of proximal drive sleeve 42 has four arms or fingers 48. A hook-like bearing surface 49 exists on the underside (as seen in Figure 10) of flange segments on the end of the flexible fingers 48. The flexible fingers 48 are separated with gaps or slots that make space for the button 70 to snap to the clutch 90 and also enable these fingers to flex inwards during assembly of the proximal drive sleeve 42 to the dial sleeve 62. After assembly the hooks 49 retain the proximal drive sleeve 42 relative to the dial sleeve 62 under the reaction force from the spring 103. During dispense the button 70 depresses the spring 103 via the clutch 90 and the clicker components and this spring 103 is reacted through the coupler 43 to the proximal drive sleeve 42 which then through these bearing surfaces applies axial load to the dial sleeve 62. This axial load drives the dial sleeve 62 and hence number sleeve 61 along the helical thread of the inner body 20, back into the body of the device, until the 0U stop faces on the number sleeve 61 contact the inner body 20.

The coupler 43 shown in Figure 11 rotationally couples the two halves of the drive sleeve together during dialing and dispense, whilst allowing them to de-couple during reset. The coupler 43 has to also transfer the last dose protection stop load from the proximal drive sleeve 42 to the distal drive sleeve 41. Two sets of teeth are provided in the coupler 43 for engaging teeth 46 and teeth 47, respectively. The coupler 43 is snapped onto distal drive sleeve 41 allowing limited relative axial movement with respect to the proximal drive sleeve 42.

The nut 50 is provided between the inner body 20 and the distal drive sleeve 41 of driver 40. Stop faces 51 are located on the proximal face of last dose nut 50 to limit the number of units that can be dialed if the stop faces 51 contact stops 45 of distal drive sleeve 41. The function of the last dose nut 50 is to prevent the user from dialing beyond a finite amount. This limit is based on the dispensable volume of the cartridge 81 and when reached, the user must replace the cartridge 81 and reset the device.

External ribs 52 of the nut 50 engage splines 22 of inner body 20. An internal thread 53 of the nut engages the external thread 44 of distal drive sleeve 41. As an alternative, splines and ribs could be provided on the interface between the nut 50 and the driver 40 and threads could be provided on the interface between the nut 50 and the inner body 20. As a further alternative, the nut 50 may be designed as e.g. a half nut.

The display member 60 is a generally tubular element which is composed of number sleeve 61 and dial sleeve 62 which are snapped together during assembly to axially and rotationally constrain these two components, which thus act as a single part.

The main functions of the number sleeve 61 depicted in Figure 8 are to provide a surface onto which dose numbers can be printed to display the dialed dose, to guide the helical path of the internal mechanism during dialing to follow the helical thread form on the piston rod 30 when threaded to the inner body 20 and to attach to the dial sleeve 62.

The number sleeve 61 is designed to be fully enclosed in the outer body 10 during dialing and dispense and therefore only the dialed dose is visible to the user through the window aperture. The number sleeve has a 0U (minimum dose) stop face 63 to limit its travel when dialed in but the 80U (maximum dose) stop faces that limit the dialed out condition are located on the dial sleeve 62. At the end of each dispense stroke, this stop face 63 engages with mating surface 25 on the inner body 20 to limit the rotational position of the number sleeve 61.

Figures 25a and 25b each show a cross section of the metal element 132 and the plastic element 133 of the outer housing part 10 showing a stop feature 140. The stop feature 140 may be an '80Units stop' feature. In particular, the stop feature 140 may be a rotational stop. The stop feature 140 is arranged on the plastic element.

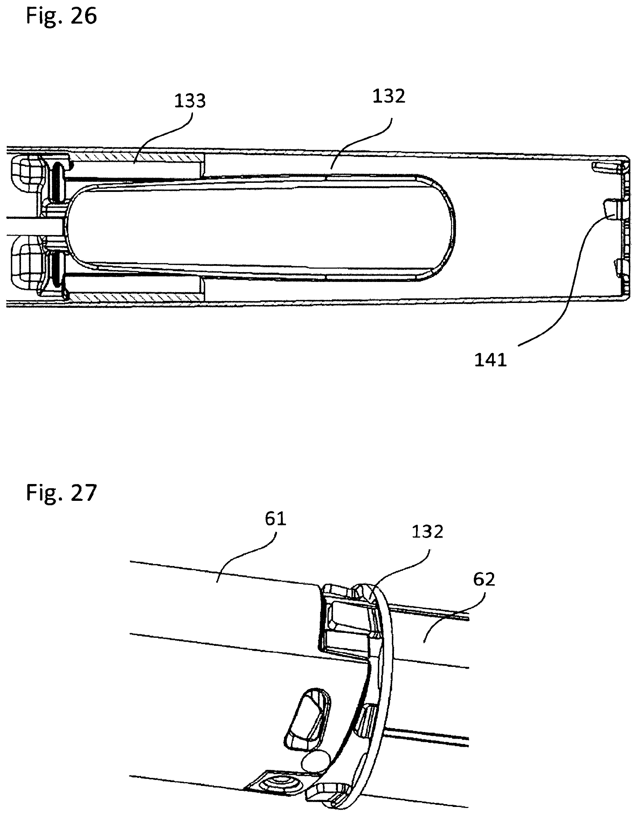

Figure 26 shows an embodiment wherein the stop feature 140 is comprised by the metal element 132 of the outer housing part 10. The stop feature 140 is formed as a series of lug features 141. The folded lug features 141 form a bearing surface for the dial sleeve 62. This bearing is advantageously of low friction to minimize dispense force and to avoid scratching the dial sleeve 62.

The advantage of the embodiment shown in Figure 26 is that the plastic element is much shorter and is therefore easier and cheaper to mold. In particular, the length of the plastic element 133 is about one third of the length of the metal element.

Furthermore an '80Units torque' is transferred via the inner housing 20 to the plastic element 133 if the user counteracts this torque by holding the cartridge holder.

Figure 27 shows an assembly with the dial sleeve 62 and the number sleeve 61 and a part of the metal element 132 comprising the stop feature 140, in particular the '80Units stop' feature. The main surface of the metal element 132 is cut away for clarity reasons. The assembly is shown in a state where an '80Units stop' feature on the dial sleeve 62 is engaged with the stop feature 140 of the metal element. By means of the stop feature 140, a rotational stop is provided for the dial sleeve 62.

A helical drive face 64 forms a thread that guides the number sleeve 61 during dialing and dispense to follow the helical path 21 on the inner body.

The dial sleeve 62 is assembled to the number sleeve 61 such that once assembled, no relative movement is allowed. The parts are made as separate components to enable both molding and assembly. Also, whereas the number sleeve 61 is preferably white to give contrast for the e.g. black dose numbers, the dial sleeve 62 color can be chosen to suit the aesthetics or perhaps to distinguish the drug type.

At the dose proximal end, the dial sleeve 62 has internal clutch features 65 that engage with the clutch component 90 during dialing and disengage from the clutch during dispense. These clutch features 65 rotationally lock the dial sleeve 62 to the clutch 90 during dialing and when the 0U and 80U stops are engaged. When the button 70 is depressed these clutch features disengage to allow the clutch 90 and drive mechanism to move axially whilst the dial sleeve 62 and number sleeve 61 spin back to the 0U start position.

The dial sleeve 62 rotates out during dialing through its engagement with the clutch 90 and number sleeve 61, and rotates back in during dispense under the axial force applied by the proximal drive sleeve 42 to a flange-like bearing face 66 on the end of the dial sleeve. This bearing face 66 engages with the flexible arms 48 of the proximal drive sleeve 42 during dispense. Two diametrically opposite faces 67 engage with the outer body 10 when the maximum dose (e.g. 80U) has been dialed, forming the maximum dose stop faces.

A ratchet arm 68 engages with ratchet features on the button 70 (dose dial grip) to provide audible feedback during dispense, giving one click per unit delivered. Further, this prevents the user from gripping and rotating the number sleeve 61 outwards from a partially dialed out position whilst holding the button 70 pressed in. This would back wind the piston rod 30 which would result in an under dose on the subsequent dialed dose. It may further strengthen the 0U stop.

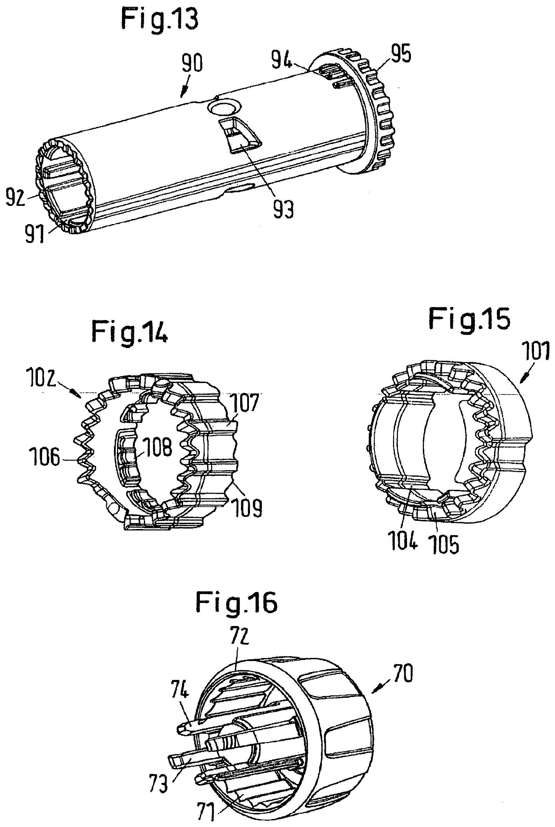

The button 70 which is shown in Figure 16 serves as a dose dial grip and is retained by the clutch 90 to transfer the actions of the user to the clutch. It also carries ratchet teeth 71 that engage the ratchet arm 68 on the dial sleeve 62, which serves as the dispensing clicker giving audible feedback (ratchet clicks), and an end face 72 which serves as the dose completion stop face with the outer body 10. This end face 72 thus serves to define the end position during dispense when it contacts the outer body 10 to provide a very positive stop improving dose accuracy.

A central sleeve-like portion of button 70 is provided with four arms 73 having hook-like snap features 74 at their respective distal ends. The arms 73 form splined surfaces engaging with the clutch 90 to transfer torque from the button 70 through the clutch to the dial sleeve 62 and proximal drive sleeve 42. The snap features 74 engage apertures in the clutch 90 and are designed with angled undercut faces to maintain engagement when an axial load is applied to pull the button 70 out of the pen body 10. The space between arms 73 defines pockets giving clearance for the flexible arms 48 of proximal drive sleeve 42 to slide freely relative to the button 70 and clutch 90 when the button 70 is depressed and released during dose dispense.

The cartridge holder 80 attaches to the inner body 20 with a bayonet connection 82 and houses the glass ampoule or cartridge 81 containing the medication to be dispensed. The cartridge holder 80 includes an aperture 83 in the rear face (as seen in Figure 6) which if gripped by the user prevents the ampoule from falling out when the cartridge holder is removed from the inner body 20. The front face is printed with a dose number scale. The threaded distal end 84 is used to attach disposable pen needles.

A tubular clutch 90 is provided between the display member 60 and the button 70. The clutch is fixed relative to and retains the button 70 and together they travel axially relative to the proximal drive sleeve 42 when the button 70 is depressed during dispense, disengaging the clutch teeth from the dial sleeve 62. It also transfers torque from the button to the proximal drive sleeve 42, and the dialing and 0U/80U stop loads from the button via the clutch teeth to the dial sleeve and number sleeve.

Drive sleeve splines 91 provided on an inner surface of the clutch engage with the proximal drive sleeve 42. At the distal end face, clutch biasing teeth 92 are provided which mate with similar teeth on the proximal clicker part 102 to ensure that in the button out position (dialed dose) the clutch is locked in rotation to the proximal clicker part 102 under the biasing action of the clutch spring 103. The teeth 92 are shallow in height to prevent the proximal clicker part 102 from engaging with splines on the proximal drive sleeve 42 during dialing. Four snap apertures 93 serve to retain the snap features 74 of button 70. Near its proximal end, the clutch has splines 94 which at the end of dispense with the button 70 depressed lock to the inner body 20 to prevent the user from rotating the button 70 below the 0U position.

Clutch teeth 95 engage with clutch teeth 65 of the dial sleeve to rotationally couple the button 70 via the clutch to the number sleeve 61. During dispense the clutch is moved axially so as to disengage these clutch teeth 95 releasing the dial sleeve 62 to rotate back into the device whilst the clutch 90 and hence driver 40 move axially to dispense the dose.

The clicker 100 comprises a distal clicker part 101, a proximal clicker part 102 and a spring 103. The clutch spring 103 serves to bias the button 70 out so that at the end of a dose the button 70 pops out, re-engaging the clutch 90 with the dial sleeve 62 ready for dialing. Further, it provides the spring force for the clicker components to act as clickers and also as detent positions for the number sleeve 61. In addition, it holds the two halves of the drive sleeves 41, 42 in rotational engagement during dialing and dispense, whilst allowing them to disengage during device reset.

The distal clicker part 101 is permanently splined to the proximal drive sleeve 42 and engages with the proximal clicker part 102 which in turn is splined to the inner body 20. During dialing when the drive sleeve is rotated relative to the inner body, the two clickers 101, 102, rotate relative to each other under the compression force of the clutch spring 103. This force combined with the clicker teeth formed on the end face of each clicker provides the clicks and also the detent dialing positions.

During dispense the two clickers 101, 102 are pressed together under the dispense load and therefore prevent relative rotation between the proximal drive sleeve 42 and inner body 20, driving the piston rod forwards to deliver the dose. The splines 104 on the inner bore rotationally couple the distal clicker part 101 to the proximal drive sleeve 42 at all times, but allow free axial movement when the button 70 is depressed during dispense and when the two clickers ride over each other during dialing. The profile of the clicker teeth 105, 106 on both distal clicker part 101 and proximal clicker part 102 are identical and ride over each other under the compressive load from the spring 103 during dialing.

The proximal clicker part 102 is permanently splined to the inner body 20 by external splines 107 which prevent relative rotation with the inner body during both dialing and dispense, providing clicks during dialing and locking the proximal drive sleeve 42 in rotation during dispense. Additional cylindrically shaped splines 108 also couple the proximal clicker part 102 rotationally to the proximal drive sleeve 42 when the button 70 is depressed, this preventing the user from dialing past 80 units with the button depressed. Proximal clicker part 102, in addition to the primary clicker teeth 106, has clutch biasing teeth 109 on the opposite end face. These teeth mate with similar teeth 92 on the clutch to ensure that in the button out position (dialed dose) the clutch is locked in rotation to the proximal clicker part 102 under the biasing action of clutch spring 103.

The cartridge bias spring 110 is assembled as two components one after the other, the lower first and the upper second. The spring combination serves to apply an end load to the cartridge 81 at extremes of tolerance so as to bias it forwards onto the end face of the ferrule in the cartridge holder 80. This ensures that when the user removes and attaches a needle, the friction between the needle cannula and septum of the cartridge does not move the cartridge 81 axially relative to the cartridge holder 80. The bias spring 110 also acts to provide a force against which the user has to connect the cartridge holder 80 and this may add to the tactile feedback of this bayonet joint. The spring 100 also serves to eject the cartridge holder 80 if the cartridge holder is not rotated into a secure position, highlighting this error to the user.

The cap 120 serves to protect the cartridge holder 80 from damage and the cartridge 81itself from dust dirt ingress on to the area around the septum. The cap is designed to accommodate a standard pen injector needle.

The window insert 130 may include a lens to magnify the dose numbers e.g. by approximately 25% from their printed size. The window insert 130 may be back printed to protect the printed surface from abrasion and also to maximize the light entering through the window aperture, giving uniform illumination of the dose numbers and white area around these numbers. Arrows may be printed adjacent to the window aperture that indicate the dose dialed.

In the following, the function of the drug delivery device and its components will be explained in more detail with reference to Figures 17 to 19.

To use the device, a user has to select a dose. In the start (at rest) condition as shown in Figure 17 the display member 60 indicates the number of doses dialed to the user. The number of dialed units can be viewed through the dose window 130 in the outer body 10. Due to the threaded engagement between the display member 60 and the inner body 20 rotation of the button 70 in a clockwise fashion causes the display member 60 to wind out of the device and incrementally count the number of units to be delivered. Figure 18 shows an intermediate stage of dialing (e.g. 7 of 80 units).

During dose setting button 70, driver 40 and display member 60 are rotationally locked together via clutch 90. Further, button 70, driver 40 and display member 60 are axially coupled. Thus, these three components wind out of the outer housing 10 during dose setting. Clockwise rotation of the button 70 causes the driver 40 to rotate and in doing so it advances along the piston rod 30 which remains fixed throughout dialing. The clicker arrangement 100 provides tactile and audible feedback to the user when dialing doses. At the maximum settable dose of 80 units, the stop features 12 and 67 engage to prevent further dialing.

The last dose nut 50 provides the function of counting the number of dispensed units. The nut 50 locks the device at the end of cartridge life and as such no more drug can be dialed by the user. The last dose nut 50 and the driver 40 are connected via a threaded interface as explained above. Further, the last dose nut 50 is assembled into splines 22 such that the nut 50 and the inner body 20 are rotationally locked together (at all times). Rotation of the driver 40 during dialing causes the nut 50 to advance along the thread 44. The nut 50 is free to slide axially within the inner body 20 at all times which allows advancement of the nut. The change in pitch of thread 44 shown in Figure 9 towards the final doses axially accelerates the advancement of the nut 50 towards the end of cartridge life lockout condition. At the end of life condition, the stop features 51 of the last dose nut 50 contact the corresponding features 45 on the driver 40. The splined contact with inner body 20 reacts any torque transmitted by these stop features 45.

With the desired dose dialed, the device 1 is ready for dose dispensing. This basically requires pushing button 70 which will result in a disengagement of the clutch 90 from dial sleeve 62 thus allowing relative rotation between the display member 60 and the button 70. In all conditions the driver 40 and the button 70 are rotationally locked together by engagement of arms 73 and fingers 48 and by splines 91 engaging corresponding splines on proximal drive sleeve 42. Thus, with the clutch 90 disengaged (button 70 pushed in) button 70 and driver 40 are rotationally locked together with the button 70, the driver 40 and the display member 60 still being axially coupled.

When dispensing a dose, the dose button 70 and clutch 90 are moved axially relative to the mechanism compressing the clutch spring 103. Because the proximal clicker part 102 is splined to the inner body 20 and the axial load passing through the clicker teeth 105, 106 locks the distal clicker part 101 in rotation to the proximal clicker part 102, the mechanism is forced to move axially whilst the dial sleeve 62 and number sleeve 61 are free to spin back into the outer housing 10. The interaction of mating threads between the piston rod 30, driver 40 and inner body 20 delivers a mechanical advantage of 2:1. In other words, axially advancing driver 40 causes the piston rod 30 to rotate which due to the threaded engagement of piston rod 30 with the inner body 20 advances the piston rod. During dose dispensing dispense clicker 68, 71 is active which involves button 70 and display member 60. The dispense clicker provides primarily audible feedback to the user that drug is being dispensed.

The end of this step is shown in Figure 19. At this point the dose is complete and when the user removes the force from the end of the dose button 70, the clutch spring 103 pushes this dose button 70 rearwards, re-engaging the teeth 65 and 95 between the clutch and the dial sleeve.

Resetting the device starts with removal of the cartridge holder 80 and replacing an empty cartridge with a full cartridge 81. As the cartridge holder is re-attached, the bung of the new cartridge contacts bearing 31, thus pushing piston rod 30 back into the housing. Initially, the piston rod 30 screws into the inner body 20, thereby axially disengaging the coupler 43 from the proximal drive sleeve 42 against the biasing force of spring 103. Once disengaged the coupler 43 is free to start rotating together with distal drive sleeve 41 and continues to do so as the cartridge holder 80 is moved axially into engagement with the inner body 20.Thus, the distal drive sleeve 41 rotates with respect to the proximal drive sleeve 42 which is still rotationally constrained in inner body 20 as clicker parts 101 and 102 are pressed together by compressed spring 103. As the distal drive sleeve 41 rotates, last dose nut 50 is reset to its (distal) start position. Coupling the cartridge holder 80 to inner body 20 backs off the mechanism due to the bayonet structure 23 allowing re-engagement of the proximal drive sleeve 42 with coupler 43 and thus the distal drive sleeve 41.

### List of references

- 1: drug delivery device
- 10: outer housing part
- 11: distal part
- 12: rotational hard stop
- 13: aperture
- 20: inner housing
- 21: external thread
- 22: splines
- 23: bayonet features
- 24: window retention nose
- 25: first rotational stop
- 30: piston rod
- 31: bearing
- 32: first outer thread
- 33: second outer thread
- 40: driver
- 41: distal driver part
- 42: proximal driver part component
- 43: coupler
- 44: external thread
- 45: stop faces
- 46: splines
- 47: teeth features
- 48: fingers
- 49: bearing surface
- 50: dose nut
- 51: stop faces
- 52: external ribs
- 53: internal thread
- 60: display member
- 61: number sleeve
- 62: dial sleeve
- 63: stop face
- 64: helical drive face
- 65: clutch features/teeth
- 66: bearing face
- 67: opposite faces
- 68: ratchet arm
- 70: button
- 71: ratchet teeth
- 72: end face
- 73: arms
- 74: snap features
- 80: cartridge holder
- 81: cartridge
- 82: bayonet connection
- 83: aperture
- 84: distal end
- 90: clutch
- 91: drive sleeve splines
- 92: clutch biasing teeth
- 93: snap features
- 94: splines
- 95: clutch teeth
- 100: clicker
- 101: distal clicker part
- 102: proximal clicker part
- 103: spring
- 104: splines
- 105, 106: clicker teeth
- 107: external splines
- 108: shaped splines
- 109: clutch biasing teeth
- 110: spring
- 120: cap
- 130: window
- 131: plastic element of cap
- 132: metal element of outer housing part
- 133: plastic element of outer housing part
- 134: clip element
- 135: aperture of cap
- 136: aperture of outer housing part
- 137: end face (of cap)
- 138: radial lug
- 139: snap feature
- 140: stop feature
- 141: lug feature stop feature
- 142: tab
- 150: metal element of cap

## Claims

1. Assembly for a drug delivery device, comprising a metal element (150, 132) and a plastic element (131, 133) which together form a functional unit, wherein the metal element (150, 132) and the plastic element (131, 133) are fixedly coupled to each other, wherein the metal element (150, 132) provides an outer surface of the assembly which is configured to be handled by a user, and wherein an outer surface of the plastic element (131, 133) is continuously adapted to an inner surface of the metal element (150, 132),
the assembly comprising an aperture (13, 135, 136),
**characterized in that**
the metal element (150, 132) and the plastic element (131, 133) are secured with respect to each other by a further component being received in the aperture (13, 135, 136), and wherein the further component is a window component (130) which is configured to indicate an amount of a set dose to the user.

2. Assembly according to claim 1, wherein the further component is clipped in the plastic element (131, 133) or wherein the further component is comprised by the plastic element (131, 133).

3. Assembly according to any of the previous claims, wherein the metal element (150, 132) and the plastic element (131, 133) have the shape of a sleeve.

4. Assembly according to any of the previous claims, wherein the metal element (150, 132) is deep drawn.

5. Assembly according to any of the previous claims, wherein the metal element (150, 132) comprises aluminium.

6. Assembly according to any of the previous claims, wherein the metal element (150, 132) has a wall thickness of 0.3 mm to 0.6 mm.

7. Assembly according to any of the previous claims, wherein the plastic element (131, 133) is produced by mold flowing.

8. Assembly according to any of the previous claims, wherein the assembly is a housing (10) or a cap (120) for a drug delivery device.

9. Assembly according to any of the previous claims, wherein the metal element (150, 132) and the plastic element (131, 133) are additionally held together by press fitting or bonding.

10. Assembly according to any of the previous claims, wherein the metal element (150, 132) and the plastic element (131, 133) are held together by a radial lug (138) of the metal element (150, 132) being engaged with the plastic element (131, 133).

11. Assembly according to any of the previous claims, wherein the plastic element (131, 133) and/or the metal element (150, 132) comprise one or more stop features (140, 141) that are configured to prevent a user from setting a dose greater than a pre-determined maximum dose.

12. Assembly according to any of the previous claims, wherein the metal element (150, 132) and the plastic element (131, 133) each comprise an aperture (135, 136) which are aligned with each other, wherein the further component is attached to the assembly through the aperture (135, 136).

13. Assembly according to any of the previous claims, wherein the metal element (150, 132) comprises the aperture (135, 136) and wherein the plastic element (131, 133) is made from a transparent material.

14. Drug delivery device (1) comprising an assembly according to any of the previous claims, and having a weight less than 30 g.

15. Method of producing an assembly according to any of claims 1 to 13, wherein the metal element (150, 132) is slid over the plastic element (131, 133) in a longitudinal direction.

## Patentansprüche

1. Baugruppe für eine Medikamenten-Verabreichungsvorrichtung, umfassend ein Metallelement (150, 132) und ein Kunststoffelement (131, 133), die zusammen eine Funktionseinheit bilden, wobei das Metallelement (150, 132) und das Kunststoffelement (131, 133) fest aneinander gekoppelt sind, wobei das Metallelement (150, 132) eine Außenfläche der Baugruppe bereitstellt, die dazu ausgelegt ist, von einem Benutzer gehandhabt zu werden, und wobei eine Außenfläche des Kunststoffelements (131, 133) durchgehend an eine Innenfläche des Metallelements (150, 132) angepasst ist, wobei die Baugruppe eine Öffnung (13, 135, 136) umfasst,
**dadurch gekennzeichnet, dass**
das Metallelement (150, 132) und das Kunststoffelement (131, 133) durch eine weitere Komponente, die in der Öffnung (13, 135, 136) aufgenommen ist, relativ zueinander gesichert sind, und wobei die weitere Komponente eine Fensterkomponente (130) ist, die dazu ausgelegt ist, dem Benutzer einen Betrag einer eingestellten Dosis anzuzeigen.

2. Baugruppe nach Anspruch 1, wobei die weitere Komponente in das Kunststoffelement (131, 133) eingeklipst ist oder wobei die weitere Komponente von dem Kunststoffelement (131, 133) umfasst ist.

3. Baugruppe nach einem der vorstehenden Ansprüche, wobei das Metallelement (150, 132) und das Kunststoffelement (131, 133) die Form einer Hülse aufweisen.

4. Baugruppe nach einem der vorstehenden Ansprüche, wobei das Metallelement (150, 132) tiefgezogen ist.

5. Baugruppe nach einem der vorstehenden Ansprüche, wobei das Metallelement (150, 132) Aluminium umfasst.

6. Baugruppe nach einem der vorstehenden Ansprüche, wobei das Metallelement (150, 132) eine Wandstärke von 0,3 mm bis 0,6 mm aufweist.

7. Baugruppe nach einem der vorstehenden Ansprüche, wobei das Kunststoffelement (131, 133) durch Formguss hergestellt ist.

8. Baugruppe nach einem der vorstehenden Ansprüche, wobei die Baugruppe ein Gehäuse (10) oder eine Kappe (120) für eine Medikamenten-Verabreichungsvorrichtung ist.

9. Baugruppe nach einem der vorstehenden Ansprüche, wobei das Metallelement (150, 132) und das Kunststoffelement (131, 133) zusätzlich durch Presspassung oder Verklebung zusammengehalten werden.

10. Baugruppe nach einem der vorstehenden Ansprüche, wobei das Metallelement (150, 132) und das Kunststoffelement (131, 133) durch eine radiale Nase (138) des Metallelements (150, 132), die mit dem Kunststoffelement (131, 133) in Eingriff steht, zusammengehalten werden.

11. Baugruppe nach einem der vorstehenden Ansprüche, wobei das Kunststoffelement (131, 133) und/oder das Metallelement (150, 132) ein oder mehrere Stoppelemente (140, 141) umfassen, die dazu ausgelegt sind, zu verhindern, dass ein Benutzer eine Dosis einstellt, die größer als eine vorgegebene Höchstdosis ist.

12. Baugruppe nach einem der vorstehenden Ansprüche, wobei das Metallelement (150, 132) und das Kunststoffelement (131, 133) jeweils eine Öffnung (135, 136) umfassen, die miteinander ausgerichtet sind, wobei die weitere Komponente durch die Öffnung (135, 136) an der Baugruppe befestigt ist.

13. Baugruppe nach einem der vorstehenden Ansprüche, wobei das Metallelement (150, 132) die Öffnung (135, 136) umfasst und wobei das Kunststoffelement (131, 133) aus einem transparenten Material hergestellt ist.

14. Medikamenten-Verabreichungsvorrichtung (1), die eine Baugruppe nach einem der vorstehenden Ansprüche umfasst und ein Gewicht von weniger als 30 g aufweist

15. Verfahren zur Herstellung einer Baugruppe nach einem der Ansprüche 1 bis 13, wobei das Metallelement (150, 132) in einer Längsrichtung über das Kunststoffelement (131, 133) geschoben wird.

## Revendications

1. Ensemble pour dispositif d'administration de médicament, comprenant un élément métallique (150, 132) et un élément en plastique (131, 133) qui forment ensemble une unité fonctionnelle, l'élément métallique (150, 132) et l'élément en plastique (131, 133) étant accouplés fixement l'un à l'autre, l'élément métallique (150, 132) constituant une surface externe de l'ensemble configurée pour être manipulée par un utilisateur, et une surface externe de l'élément en plastique (131, 133) étant adaptée en continu à une surface interne de l'élément métallique (150, 132), l'ensemble comprenant une ouverture (13, 135, 136),
**caractérisé en ce que**
l'élément métallique (150, 132) et l'élément en plastique (131, 133) sont fixés l'un par rapport à l'autre par un composant supplémentaire reçu dans l'ouverture (13, 135, 136), et le composant supplémentaire étant un composant de fenêtre (130) configuré pour indiquer à l'utilisateur une quantité d'une dose définie.

2. Ensemble selon la revendication 1, le composant supplémentaire étant clipsé dans l'élément en plastique (131, 133) ou le composant supplémentaire étant constitué par l'élément en plastique (131, 133).

3. Ensemble selon l'une quelconque des revendications précédentes, l'élément métallique (150, 132) et l'élément en plastique (131, 133) ayant la forme d'un manchon.

4. Ensemble selon l'une quelconque des revendications précédentes, l'élément métallique (150, 132) étant embouti.

5. Ensemble selon l'une quelconque des revendications précédentes, l'élément métallique (150, 132) comprenant de l'aluminium.

6. Ensemble selon l'une quelconque des revendications précédentes, l'élément métallique (150, 132) ayant une épaisseur de paroi de 0,3 mm à 0,6 mm.

7. Ensemble selon l'une quelconque des revendications précédentes, l'élément en plastique (131, 133) étant produit par écoulement de moule.

8. Ensemble selon l'une quelconque des revendications précédentes, l'ensemble étant un logement (10) ou un capuchon (120) destiné à un dispositif d'administration de médicament.

9. Ensemble selon l'une quelconque des revendications précédentes, l'élément métallique (150, 132) et l'élément en plastique (131, 133) étant en outre maintenus ensemble par emmanchement à force ou collage.

10. Ensemble selon l'une quelconque des revendications précédentes, l'élément métallique (150, 132) et l'élément en plastique (131, 133) étant maintenus ensemble par une patte radiale (138) de l'élément métallique (150, 132) en prise avec l'élément en plastique (131, 133).

11. Ensemble selon l'une quelconque des revendications précédentes, l'élément en plastique (131, 133) et/ou l'élément métallique (150, 132) comprenant un ou plusieurs éléments de butée (140, 141) configurés pour empêcher un utilisateur de régler une dose supérieure à une dose maximale prédéterminée.

12. Ensemble selon l'une quelconque des revendications précédentes, l'élément métallique (150, 132) et l'élément en plastique (131, 133) comprenant chacun une ouverture (135, 136) qui sont alignées l'une avec l'autre, le composant supplémentaire étant fixé à l'ensemble à travers l'ouverture (135, 136).

13. Ensemble selon l'une quelconque des revendications précédentes, l'élément métallique (150, 132) comprenant l'ouverture (135, 136) et l'élément en plastique (131, 133) étant réalisé en un matériau transparent.

14. Dispositif d'administration de médicament (1) comprenant un ensemble selon l'une quelconque des revendications précédentes, et ayant un poids inférieur à 30 g.

15. Procédé de fabrication d'un ensemble selon l'une quelconque des revendications 1 à 13, l'élément métallique (150, 132) étant glissé sur l'élément en plastique (131, 133) dans une direction longitudinale.
